Europäisches Patentamt

European Patent Office

Office européen des brevets

(11) Veröffentlichungsnummer: **0 313 685 B1**

(12) **EUROPÄISCHE PATENTSCHRIFT**

(45) Veröffentlichungstag der Patentschrift: **26.08.92**

(21) Anmeldenummer: **87118833.0**

(22) Anmeldetag: **18.12.87**

(51) Int. Cl.5: **C12M 1/16**, C12M 1/14

(54) **Verfahren zum Ernten von vermehrten Organismen sowie Festmedium-Fermenter zur Durchführung des Verfahrens.**

(30) Priorität: **28.10.87 DE 3736571**

(43) Veröffentlichungstag der Anmeldung:
**03.05.89 Patentblatt 89/18**

(45) Bekanntmachung des Hinweises auf die
Patenterteilung:
**26.08.92 Patentblatt 92/35**

(84) Benannte Vertragsstaaten:
**CH DE FR GB IT LI**

(56) Entgegenhaltungen:
**DE-C- 305 198**
**FR-A- 819 173**
**GB-A- 758 811**
**GB-A- 984 404**

(73) Patentinhaber: **Infors GmbH**
**Aidenbachstrasse 144a**
**W-8000 München 71(DE)**

(72) Erfinder: **Hawrylenko, Alexander**
**Ahornstr. 5**
**CH-4103 Bottmingen(CH)**

(74) Vertreter: **Säger, Manfred, Dipl.-Ing.**
**Säger & Partner Patentanwälte Postfach 81**
**08 09**
**W-8000 München(DE)**

## Beschreibung

Die Erfindung betrifft ein Verfahren zur Züchtung von Organismen, z.B. Bakterien, Hefen, Pilze und Zellen und/oder zur Herstellung von Produkten, die durch diese erzeugt werden, sowie einen Festmedium-Fermenter zur Durchführung des Verfahrens.

Bei bekannten Verfahren wird ein Träger beispielsweise als rundes Substrat sterilisiert, mit einem Nährboden versehen und dann mit Organismen geimpft. Dieser Träger wird dann manuell in einen Brutkasten gebracht, wo die Vermehrung stattfindet. Danach wird der plattenförmige Träger entnommen und die vermehrten Organismen beispielsweise durch Abbürsten abgeerntet.

Von Nachteil bei diesem bekannten Verfahren ist das diskontinuierliche Ablaufen und die vielen manuellen Arbeitsgänge, die eine Vermehrung mit gutem Wirkungsgrad nicht zulassen.

Der Erfindung liegt deshalb die Aufgabe zugrunde, ein gattungsgemäßes Verfahren sowie eine Vorrichtung zur Durchführung des Verfahrens bereitzustellen, mit denen eine effizientere Vermehrung von Organismen möglich ist.

Diese Aufgabe wird bei einem gattungsgemäßen Verfahren sowie einer gattungsgemäßen Vorrichtung erfindungsgemäß durch die kennzeichnenden Merkmale des Verfahrens- sowie Vorrichtungs-Hauptanspruchs gelöst.

Erfindungsgemäß wird also ein kontinuierliches Vermehrungsverfahren auf einem Band als Träger durchgeführt. Das mit Nährboden versehene Band, auf den die Organismen aufgebracht werden, durchläuft dann die Inkubationseinheit als zweite Station, in der vorzugsweise das endlose Band über eine Vielzahl von zueinander parallelen Umlenkrollen vorzugsweise mäanderförmig geführt ist. In dieser zweiten Station wird ferner die zum Vermehren erforderliche Temperatur durch Beheizen aufgebracht. Das am Ende austretende Band wird dann in der dritten Station kontinuierlich abgeerntet und die abgeernteten Organismen abtransportiert und gegebenenfalls an anderer Stelle gesammelt. Mit der Erfindung läßt sich in ökonomischer Weise kontinuierlich eine Vermehrung von Organismen durchführen.

Zweckmäßige Ausgestaltungen und Weiterbildungen der Erfindung sind in den Unteransprüchen gekennzeichnet.

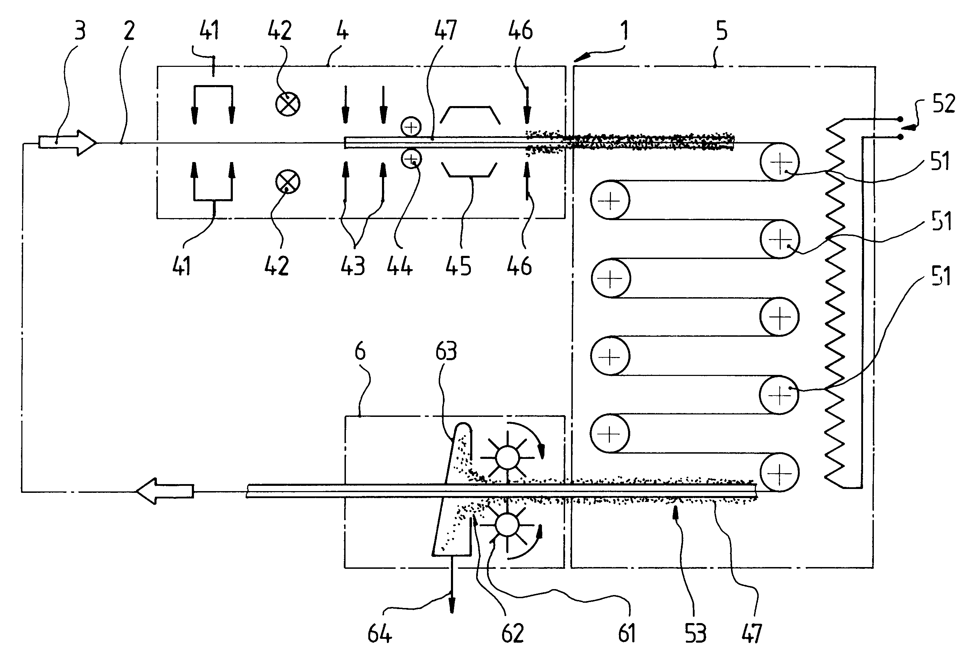

Ein bevorzugtes Ausführungsbeispiel der Erfindung wird nachfolgend unter Bezugnahme auf die Zeichnung näher erläutert, die einen schematischen Querschnitt durch einen Festmedium-Fermenter als Vorrichtung zur Durchführung des Verfahrens zeigt.

Der insgesamt mit 1 bezeichnete Fermenter weist ein endlos umlaufendes Band 2 als Träger auf, welches vorzugsweise aus Kunststoff besteht. In schematisch mit dem Pfeil 3 bezeichneten Laufrichtung durchläuft das Band 2 drei hintereinander angeordnete Stationen, nämlich eine erste Station 4, eine zweite Station 5 und schließlich eine dritte Station 6. Danach wird das Band 2 wieder, wie schematisch angedeutet, zu dem Eingang der ersten Station 4 geführt.

In der ersten Station 4 sind in Laufrichtung 3 des Bandes 2 zunächst schematisch mit 41 bezeichnete Sterilisationsdüsen für Dampf und/oder Gas dargestellt. Zusätzlich können noch UV-Lampen 42 zum Sterilisieren vorgesehen sein.

Ferner sind in der ersten Station 4 Düsen oder Auftragsdüsen 43 zum Auftragen eines Nährmediums oder Nährbodens 47 auf das Band 2 vorgesehen. Zusätzlich können Walzen oder Auftrags- und/oder Anpreßwalzen 44 vorgesehen sein. Dahinter kann eine insgesamt mit 45 bezeichnete Heizvorrichtung zum Trocknen des Nährmediums 47 vorgesehen sein. In Laufrichtung 3 dahinter sind Impfdüsen 46 zum Aufbringen von Organismen auf den schematisch mit 47 bezeichneten Nährboden angeordnet. Das solchermaßen präparierte Band 2 läuft aus der ersten Station 4 in die zweite Station 5.

In dieser ist eine Vielzahl von zueinander parallelen Umlenkrollen 51 vorgesehen, um die das Band 2 mäanderförmig herumgeführt ist. Die zweite Station 5 weist ferner eine elektrische Heizvorrichtung 52 auf, mittels der in der zweiten Station 5 eine vorgebbare Temperatur eingestellt und gehalten werden kann.

Am Ende der zweiten Station 5 tritt das Band 2 in die dritte Station 6 über, in der die vermehrten Organismen 53 auf dem Nährboden 47 mittels schematisch mit 61 bezeichneten Bürsten und/oder Messern abgenommen werden. Die abgenommenen Organismen 62 werden mittels einer Sammelleitung 63, die an eine nicht gezeigte Unterdruckquelle 64 angeschlossen ist, abgesaugt und an anderer Stelle gesammelt.

Nach der dritten Station 6 kann das Band 2 vom Nährboden 47 befreit werden. Dies kann aber auch zu allererst in der ersten Station 4 erfolgen.

## Patentansprüche

1.  Verfahren zur Züchtung von Organismen, z.B. Bakterien, Hefe, Pilze und Zellen und/oder zur Herstellung von Produkten, die durch diese erzeugt werden, **dadurch gekennzeichnet,** daß ein intermittierend oder kontinuierlich laufendes Band (2) als Träger verwendet wird und daß dieses aufeinanderfolgend eine erste Station (4) zum Sterilisieren und Beimpfen mit Organismen, danach eine Inkubationseinheit als zweite Station (5) und schließlich eine dritte Station (6) zum Ernten der in der zweiten Station (4) vermehrten Organismen oder Produkte von dem Band (2) durchläuft und daß in der ersten Station (4) nach dem Sterilisieren und vor dem Beimpfen ein Nährmedium (47) für die Organismen oder Zellen als Festmedium auf das Band (2) aufgebracht wird.

2. Verfahren nach Anspruch 1, **dadurch gekennzeichnet,** daß das Band (2) endlos umläuft und nach der dritten Station (6) wieder zu der ersten Station (4) zurückgeführt ist.

3. Verfahren nach Anspruch 1 oder 2, **dadurch gekennzeichnet,** daß das Band (2) in der zweiten Station (5) über eine Vielzahl von Umlenkrollen (51) hin- und hergeführt sind.

4. Verfahren nach Anspruch 3, **dadurch gekennzeichnet,** daß das Band (2) mäanderförmig von den Umlenkrollen (51) geführt ist.

5. Verfahren nach einem der Ansprüche 1 bis 4, **dadurch gekennzeichnet,** daß das Nährmedium (47) mittels Düsen (43) aufgesprüht und/oder Walzen (44) aufgebracht oder verdichtet wird.

6. Verfahren nach Anspruch 4 oder 5, **dadurch gekennzeichnet,** daß das Nährmedium (47) nach dem Auftragen mittels einer Belüftungsund/ oder Heizvorrichtung (45) getrocknet wird.

7. Verfahren nach einem der Ansprüche 1 bis 6, **dadurch gekennzeichnet,** daß das Sterilisieren des Bandes (2) in der ersten Station (4) mittels Bedampfen, Begasen und/oder Bestrahlen, z.B. mit Gamma-Strahlung oder durch Beleuchten mit UV-Licht erfolgt.

8. Verfahren nach einem der Ansprüche 1 bis 7, **dadurch gekennzeichnet,** daß die vermehrten Organismen (53) oder deren Produkte in der dritten Station (6) von dem Band (2) bzw. dem Nährboden (47) getrennt, abgenommen und gegebenenfalls gesammelt werden.

9. Verfahren nach Anspruch 8, **dadurch gekennzeichnet,** daß die Organismen (53) oder deren Produkte von dem Band (2) mittels eines Messers abgeschält und/oder einer Bürste (61) und/oder chemisch gelöst und über eine Sammelleitung (63), die an eine Unterdruckquelle (64) angeschlossen ist, abgesaugt werden.

10. Verfahren nach einem der Ansprüche 1 bis 9, dadurch gekennzeichnet, daß das Band (2) in der ersten Station (4) beidseitig mit dem Nährmedium (47) versehen und beimpft wird.

11. Festmedium-Fermenter zur Durchführung des Verfahrens, insbesondere nach einem der Ansprüche 1 bis 10, gekennzeichnet durch ein als Träger dienendes Band (2), eine erste Station (4) mit einer Sterilisationseinrichtung (41, 42) für das Band (2), eine Einrichtung (43, 44) zum Auftragen eines Nährbodens oder Nährmediums (47) auf das Band (2) und mit einer Beimpfungseinrichtung (46) zum Aufbringen von Organismen auf den Nährboden (47), einer der ersten Stationen (4) nachgeschalteten und vom Band (2) durchlaufenden Inkubationseinheit als zweite Station (5) und durch eine der zweiten Station (5) nachgeschalteten und vom Band (2) durchlaufenen dritte Station (6) mit einer Ernteeinrichtung (61) zum kontinuierlichen Abernten der gezüchteten Organismen (53) und einer Transporteinrichtung (63, 64) zum Entfernen der abgeernteten Organismen (62).

12. Festmedium-Fermenter nach Anspruch 11, dadurch gekennzeichnet, daß das Band (2) endlos kontinuierlich umlaufend ausgebildet ist.

13. Festmedium-Fermenter nach Anspruch 11 oder 12, dadurch gekennzeichnet, daß die Sterilisationseinrichtung aus Sterilisationsdüsen (41) für Dampf- und/oder Gas und/oder UV-Lampen (42) besteht.

14. Festmedium-Fermenter nach einem der Ansprüche 11 bis 13, dadurch gekennzeichnet, daß die Einrichtung zum Auftragen des Nährbodens (47) aus Auftragsdüsen (43) und/oder Auftrags- und/oder Anpreßwalzen (44) besteht.

15. Festmedium-Fermenter nach einem der Ansprüche 11 bis 14, dadurch gekennzeichnet, daß die Beimpfungseinrichtung Impfdüsen (46) zum Aufbringen der Organismen aufweist.

16. Festmedium-Fermenter nach einem der Ansprüche 11 bis 15, dadurch gekennzeichnet, daß die Inkubationseinheit eine Vielzahl von zueinander parallelen Umlenkrollen (51) zur vorzugsweisen mäanderförmigen Führung des Bandes (2) und eine Heizvorrichtung (52) umfaßt.

17. Festmedium-Fermenter nach einem der Ansprüche 11 bis 16, dadurch gekennzeichnet, daß die Ernteeinrichtung aus Bürsten und/oder Messern (61) und die Transporteinrichtung aus einer an eine Unterdruck quelle (64) angeschlossene Sammelleitung (63) besteht.

18. Festmedium-Fermenter nach einem der Ansprüche 11 bis 17, dadurch gekennzeichnet, daß zwischen der Einrichtung (43, 44) zum Auftragen des Nährbodens (47) und der Beimpfungseinrichtung (46) eine Heizvorrichtung (45) zum Trocknen des Nährbodens (47) angeordnet ist.

19. Festmedium-Fermenter nach einem der Ansprüche 11 bis 18, dadurch gekennzeichnet, daß die drei Stationen (4, 5, 6) einstückig ausgebildet sind.

20. Festmedium-Fermenter nach einem der Ansprüche 11 bis 18, dadurch gekennzeichnet, daß die drei Stationen (4, 5, 6) trennbar sind.

21. Festmedium-Fermenter nach einem der Ansprüche 11 bis 20, dadurch gekennzeichnet, daß das Band (2) aus Kunststoff besteht.

**Claims**

1. A process for cultivating organisms, for example bacteria, yeast, fungi and cells and/or for the manufacture of products which are produced by same, characterised in that an intermittently or continuously moving belt (2) is used as a carrier and that it successively passes through a first station (4) for sterilization and inoculation with organisms, thereafter an incubation unit as a second station (5) and finally a third station (6) for collection from the belt (2) of the products or organisms multiplied in the second station (4) and that a nutrient medium (47) for the organisms or cells is applied in the form of a solid medium to the belt (2) in the first station (4) after the sterilisation step and prior to the inoculation step.

2. A process according to claim 1 characterised in that the belt (2) endlessly circulates and is returned to the first station (4) again after the third station (6).

3. A process according to claim 1 or claim 2 characterised in that the belt (2) is guided to and fro around a plurality of direction-changing rollers (51) in the second station (5).

4. A process according to claim 3 characterised in that the belt (2) is guided in a meander configuration by the direction-changing rollers (51).

5. A process according to one of claims 1 to 4 characterised in that the nutrient medium (47) is sprayed on by means of nozzles (43) and/or applied or compressed by means of rollers (44).

6. A process according to claim 4 or claim 5 characterised in that the nutrient medium (47) is dried after being applied by means of a ventilation and/or heating means (45).

7. A process according to one of claims 1 to 6 characterised in that sterilisation of the belt (2) is effected in the first station (4) by means of treatment with vapour, treatment with gas and/or treatment with radiation, for example gamma radiation or by illumination with UV-light.

8. A process according to one of claims 1 to 7 characterised in that the multiplied organisms (53) or the products thereof are separated from the belt (2) or the nutrient medium (47) in the third station (6), removed and possibly collected.

9. A process according to claim 8 characterised in that the organisms (53) or the products thereof are peeled from the belt (2) by means of a knife and/or a brush (61) and/or chemically released and are sucked away by way of a collecting conduit (63) which is connected to a reduced-pressure source (64).

10. A process according to one of claims 1 to 9 characterised in that in the first station (4) the belt (2) is provided on both sides with the nutrient medium (47) and inoculated.

11. A solid-medium fermenter for carrying out the process in particular according to one of claims 1 to 10 characterised by:
a belt (2) serving as a carrier,
a first station (4) having a sterilisation means (41, 42) for the belt (2), a means (43, 44) for applying a culture medium or nutrient medium (47) to the belt (2) and an incoulation means (46) for applying organisms to the culture medium (47),
an incubation unit which is disposed downstream of the first station (4) and through which the belt (2) passes, as the second station (5), and
a third station (6) which is disposed downstream of the second station (5) and through which the belt (2) passes, having a collecting means (61) for continuously collecting the cultivated organisms (53) and a transportation means (63, 64) for removal of the collected organisms (62).

12. A solid-medium fermenter according to claim 11 characterised in that the belt (2) is designed for endless continuous circulation.

13. A solid-medium fermenter according to claim 11 or claim 12 characterised in that the sterilisation means comprises sterilisation nozzles (41) for vapour and/or gas and/or UV-lamps (42).

14. A solid-medium fermenter according to one of claims 11 to 13 characterised in that the means for applying the culture medium (47) comprises applicator nozzles (43) and/or applicator and/or pressure rollers (44).

15. A solid-medium fermenter according to one of claims 11 to 14 characterised in that the inoculation means has inoculation nozzles (46) for applying the organisms.

16. A solid-medium fermenter according to one of claims 11 to 15 characterised in that the incubation unit includes a plurality of mutually parallel direction-changing rollers (51) for guiding the belt (2) preferably in a meander-like configuration, and a heating means (52).

17. A solid-medium fermenter according to one of claims 11 to 16 characterised in that the collecting means comprises brushes and/or knives (61) and the transportation means comprises a collecting conduit (63) connected to a reduced-pressure source (64).

18. A solid-medium fermenter according to one of claims 11 to 17 characterised in that a heating means (45) for drying the culture medium (47) is arranged between the means (43, 44) for applying the culture medium (47) and the inoculation means (46).

19. A solid-medium fermenter according to one of claims 11 to 18 characterised in that the three stations (4, 5, 6) are of an integral structure.

20. A solid-medium fermenter according to one of claims 11 to 18 characterised in that the three stations (4, 5, 6) are separable.

21. A solid-medium fermenter according to one of claims 11 to 20 characterised in that the belt (2) comprises plastics material.

**Revendications**

1. Procédé pour la culture d'organismes, par exemple de bactéries, de levures, de champignons et de cellules et/ou pour la préparation de produits générés par ces derniers, caractérisé par le fait qu'un tapis (2) roulant par intermittence ou en continu est utilisé comme support et que ce tapis parcourt consécutivement un premier poste (4) de stérilisation et d'inoculation d'organismes, ensuite un deuxième poste (5) constituant une unité d'incubation et enfin un troisième poste (6) pour la récolte des organismes ou produits qui se sont multipliés dans le deuxième poste (4) et par le fait que dans le premier poste (4), après la stérilisation et avant l'inoculation, est appliqué sur le tapis (2) un bouillon de culture (47) pour les organismes ou les cellules sous la forme d'un milieu solide.

2. Procédé selon la revendication 1, caractérisé par le fait que le tapis (2) tourne sans fin et après le troisième poste (6) est ramené au premier poste (4).

3. Procédé selon la revendication 1 ou 2, caractérisé par le fait que, dans le deuxième poste (5) le tapis effectue un mouvement de va-et-vient en passant sur une pluralité de poulies de renvoi (51).

4. Procédé selon la revendication 3, caractérisé par le fait que le tapis (2), sous l'effet des poulies de renvoi (51), suit un parcours en forme de méandres.

5. Procédé selon l'une des revendications 1 à 4 caractérisé par le fait que le bouillon de culture (47) est appliqué par arrosage au moyen de gicleurs (43) et/ou appliqué ou compacté au moyen de rouleaux (44).

6. Procédé selon la revendication 4 ou 5, caractérisé par le fait que le bouillon de culture (47) est séché après application au moyen d'un dispositif de ventilation et/ou de chauffage (45).

7. Procédé selon l'une des revendications 1 à 6, caractérisé par le fait que la stérilisation du tapis (2) dans le premier poste (4) s'effectue par application de vapeur, de gaz et/ou de rayons, par exemple des rayons gamma, ou par exposition à la lumière ultraviolette.

8. Procédé selon l'une des revendications 1 à 7, caractérisé par le fait que, dans le troisième poste (6), les organismes multipliés (53) ou leurs produits sont séparés du tapis (2), à savoir du bouillon de culture (47), prélevés et éventuellement collectés.

9. Procédé selon la revendication 8, caractérisé par le fait que les organismes (53) ou leurs produits sont raclés du tapis (2) au moyen d'un couteau et/ou d'une brosse (61) et/ou dissous chimiquement et qu'ils sont aspirés par une conduite collectrice (63) qui est raccordée à une source de pression négative (64).

10. Procédé selon l'une des revendications 1 à 9, caractérisé par le fait que, dans le premier poste (4), a lieu l'application du bouillon de culture et l'inoculation sur les deux faces du tapis (2).

11. Dispositif de fermentation à bouillon de culture pour la mise en oeuvre du procédé en particulier selon l'une des revendications 1 à 10, caractérisé en ce qu'il comprend :
    - un tapis (2) servant de support,
    - un premier poste (4) comportant un dispositif de stérilisation (41, 42) pour le tapis (2), un dispositif (43, 44) pour l'application d'un bouillon de culture ou d'un milieu nutritif (47) sur le tapis (2) et un dispositif d'inoculation (46) pour l'application d'organismes sur le milieu nutritif (47),
    - un deuxième poste (5) disposé après le premier poste (4) et constituant une unité d'incubation parcourue par le tapis (2), et
    - un troisième poste (6) monté après le deuxième poste (5) et parcouru par le tapis (2) contenant un dispositif de récolte (61) pour la récolte en continu des organismes cultivés (53) et un dispositif de transport (63, 64) pour l'évacuation des organismes récoltés (62).

12. Dispositif de fermentation à bouillon de culture selon la revendication 11, caractérisé par le fait que le tapis (2) est réalisé sous la forme d'un tapis sans fin tournant en continu.

13. Dispositif de fermentation selon la revendication 11 ou 12, caractérisé par le fait que le dispositif de stérilisation est constitué de gicleurs de stérilisation (41) pour vapeur et/ou gaz et/ou de lampes UV (42).

14. Dispositif de fermentation selon l'une des revendications 11 à 13, caractérisé par le fait que le dispositif pour l'application du milieu nutritif (47) est constitué de gicleurs d'application (43) et/ou de rouleaux d'application (44) et/ou de compression.

15. Dispositif de fermentation selon l'une des revendications 11 à 14, caractérisé par le fait que le dispositif d'inoculation présente des gicleurs (46) prévus à cet effet pour l'application des organismes.

16. Dispositif de fermentation selon l'une des revendications 11 à 15, caractérisé par le fait que l'unité d'incubation comprend une pluralité de poulies de renvoi (51) parallèles les unes aux autres pour permettre au tapis (2) de suivre un trajet de préférence en forme de méandres et un dispositif de chauffage (52).

17. Dispositif de fermentation selon l'une des revendications 11 à 16, caractérisé par le fait que le dispositif de récolte est constitué de brosses et/ou de couteaux (61) et que le dispositif de transport est constitué d'une conduite collectrice (63) raccordée à une source de pression négative (64).

18. Dispositif de fermentation selon l'une des revendications 11 à 17, caractérisé par le fait qu'entre le dispositif (43, 44) pour l'application du milieu nutritif (47) et le dispositif d'inoculation (46) est disposé un dispositif chauffant (45) pour le séchage du milieu nutritif (47).

19. Dispositif de fermentation selon l'une des revendications 11 à 18, caractérisé par le fait que les trois postes (4, 5, 6) sont réalisés en une seule pièce.

20. Dispositif de fermentation selon l'une des revendications 11 à 18, caractérisé par le fait que les trois postes (4, 5, 6) peuvent être séparés.

21. Dispositif de fermentation selon l'une des revendications 11 à 20, caractérisé par le fait que le tapis (2) est constitué en une matière plastique.

EP 0 313 685 B1